(19) Europäisches Patentamt
European Patent Office
Office européen des brevets

(11) EP 4 563 978 A1

(12) EUROPEAN PATENT APPLICATION

(43) Date of publication:
04.06.2025 Bulletin 2025/23

(51) International Patent Classification (IPC):
G01N 21/41 (2006.01)      G01N 21/43 (2006.01)
G01N 21/51 (2006.01)      G01N 21/85 (2006.01)
G01N 21/47 (2006.01)

(21) Application number: 23213193.8

(22) Date of filing: 30.11.2023

(52) Cooperative Patent Classification (CPC):
G01N 21/8507; G01N 21/4133; G01N 21/431;
G01N 21/51; G01N 21/474; G01N 2021/432;
G01N 2021/4709; G01N 2021/8528

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Stichting IMEC Nederland
6708 WH Wageningen (NL)

• Kho, Esther
  6709 VK Wageningen (NL)
• Wentink, Eva
  5591 MT Heeze (NL)
• Zhang, Xu
  3931 ZH Woudenberg (NL)
• Verbiest, Vera
  6721 SV Bennekom (NL)

(72) Inventors:
• Zentile, Mark
  6822 NB Arnhem (NL)

(74) Representative: Körfer, Thomas
Mitscherlich PartmbB
Patent- und Rechtsanwälte
Karlstraße 7
80333 München (DE)

(54) SYSTEM AND METHOD FOR SIMULTANEOUSLY MEASURING REFRACTIVE INDEX AND TURBIDITY

(57)    A system (100) for simultaneously measuring refractive index and turbidity of a liquid sample (101) is provided. The system comprises a coherent light source (102) configured to generate a coherent light signal (103), an optical structure (104) being in contact with the liquid sample (101), the optical structure (104) is configured to transmit the coherent light signal (103) to the liquid sample (101) and further to receive a light signal (105) from the liquid sample (101) in response to the coherent light signal (103), and a detector (106) configured to receive the light signal (105) and further to measure refractive index and turbidity of the liquid sample (101) simultaneously from the light signal (105).

100

Fig. 1

EP 4 563 978 A1

**Description**

The invention relates to the simultaneous measurement of refractive index and turbidity of a liquid sample, especially to measure a relationship between dissolved solids and undissolved solids in the liquid sample.

Generally, refractometry may be used to quantify the amount of dissolved solids in a liquid solvent, since the dissolved substances may increase the total refractive index of the solvent. On the other hand, turbidity may be used as a measure of the undissolved solids in the liquid solvent, since the undissolved solids may create a non-homogeneous medium that may cause scattering of incident light.

For example, the document CN 115236034 A discloses a reflective refractometer provided with a prism to measure the refractive index and turbidity of a liquid. However, the refractometry scheme may require a larger active area, for example, to be contacted with the surface of the liquid. Moreover, the document does not utilize the refractive index measurement and turbidity measurement in combination.

Accordingly, an object of the invention is to provide a system and a method for simultaneously measuring refractive index and turbidity of a liquid sample, which can be integrated into smaller spaces. Another object is to utilize the refractive index measurement and turbidity measurement in combination to measure a relationship between dissolved solids and undissolved solids, e.g., the ratio of dissolved to undissolved solids in the liquid sample.

These and other objects are solved by the features of the first independent claim for the system and by the features of the second independent claim for the method. The dependent claims contain further developments.

According to a first aspect of the invention, a system for simultaneously measuring refractive index and turbidity of a liquid sample is provided. The system comprises a coherent light source configured to generate a coherent light signal. The system further comprises an optical structure being in contact with the liquid sample, the optical structure is configured to transmit the coherent light signal to the liquid sample and further to receive a light signal from the liquid sample in response to the coherent light signal.

Moreover, the system comprises a detector configured to receive the light signal and further to measure refractive index and turbidity of the liquid sample simultaneously from the light signal.

Therefore, the coherent light source and the optical structure for conveying the coherent light may facilitate a much smaller active area, e.g., to be contacted with the surface of the liquid sample to measure the back reflected and/or scattered light in response to the incident coherent light, which can be integrated into smaller spaces.

Preferably, the detector is configured to measure or estimate a concentration of dissolved solids in the liquid sample from the refractive index measurement. Additionally or alternatively, the detector is configured to measure or estimate a concentration of undissolved solids in the liquid sample from the turbidity measurement.

For example, the measurement of the refractive index may be understood as a measurement of the Fresnel reflection, especially at the surface of the liquid sample. On the other hand, the measurement of turbidity may be understood as the measurement of scattered light directed from the liquid sample in the same optical mode as the Fresnel reflection, where the intensity of the combination of Fresnel reflection and scattered light fluctuates over time, dependent on the turbidity of the sample.

For example, the term estimation can be understood as an approximation of the measurements with respect to reference measurements. For instance, the refractive index and/or turbidity measurements may be correlated or compared with reference refractive index and/or turbidity measurements corresponding to known concentrations of dissolved and/or undissolved solids in a reference liquid sample.

Advantageously, the simultaneous measurements of refractive index and turbidity may be utilized in combination, e.g., to measure the ratio of dissolved to undissolved solids in the liquid sample.

Preferably, the concentration of dissolved solids in the liquid sample is a concentration of dissolved metabolites in a urine sample. In addition, the concentration of undissolved solids in the liquid sample is a concentration of secreted proteins, e.g., the fraction of undissolved proteins, in the urine sample. Additionally or alternatively, the concentration of undissolved solids may be caused by blood cells, mucus, crystals etc. in the urine sample, thereby increasing the turbidity.

This may advantageously allow for a simultaneous estimation of protein content and specific gravity (USG) of the urine sample, and their ratio can be used as a possible parameter to yield proteinuria diagnosis.

Preferably, the concentration of dissolved solids in the liquid sample is a concentration of a substrate in a bioreactor. Additionally, the concentration of undissolved solids in the liquid sample is a concentration of cells in suspension in the bioreactor.

This may advantageously allow for a simultaneous measurement of cell concentration and substrate concentration in the bioreactor, and their ratio can be used to calculate the optimal cell growth rate and/or the nutrient consumption rate in the bioreactor.

Alternatively, the concentration of dissolved solids in the liquid sample may be a concentration of dissolved sugars in a fruit juice sample. Further alternatively, the concentration of dissolved solids in the liquid sample may be a concentration of dissolved salts in a water sample.

Preferably, the light signal comprises one or more lights reflected at a surface of the liquid sample and/or one or more lights scattered within the liquid sample, especially in response to the coherent light signal. For instance, the back reflected and/or scattered lights may be in the same spatial mode of the coherent light signal.

Preferably, the optical structure comprises a single optical fiber configured to transmit the coherent light signal to the liquid sample and further to receive the light signal from the liquid sample. In this regard, the single optical fiber is a single mode optical fiber.

Preferably, the single optical fiber is configured such that an end of the single optical fiber is immersed in the liquid sample.

Advantageously, for example, a much smaller active area can be achieved by using only one single mode optical fiber, which may have a core diameter of $8\mu m$ to $10\mu m$.

Preferably, the optical structure comprises an optical circulator configured to direct the coherent light signal towards the liquid sample and further to direct the light signal from the liquid sample.

For example, the optical circulator may be a one-directional single mode fiber optic circulator, which may advantageously allow for bidirectional propagation of light in the single optical fiber.

Preferably, the system further comprises a signal generator configured to modulate the coherent light source, preferably at a predefined frequency. Advantageously, for example, a set of parameters, such as angle, power, phase and polarization, of the coherent light can be controlled.

Preferably, the system comprises an optical switch, and a plurality of optical structures. In this regard, the optical switch is configured such that each of the plurality of optical structures transmits a respective coherent light signal from the coherent light source to the liquid sample and further to receive a respective light signal from the liquid sample in response to the coherent light signal.

Moreover, the detector is configured to receive the light signals from the plurality of optical structures and further to measure refractive index and turbidity of the liquid sample simultaneously from each of the light signals.

Advantageously, for example, a plurality of single mode fibers can be multiplexed via the optical switch without needing additional light sources and detectors, which may result in a cost-effective scheme.

For example, the plurality of single mode fibers can be used for a single liquid sample, e.g., to achieve multiple simultaneous measurements of the refractive index and turbidity at different surface locations of the liquid sample (e.g., for measuring a difference of the measurements at different locations) or at different timing instances (e.g., for measuring a change over time).

Alternatively, the plurality of single mode fibers can be used for a corresponding plurality of liquid samples to simultaneously measure refractive index and turbidity of the respective liquid samples.

Preferably, the detector is configured to measure a resultant reflection from the light signal corresponding to an interference between lights due to Fresnel reflection and lights from scattering in the liquid sample, especially due to the transmission of the coherent light signal to the liquid sample.

In this regard, the detector is configured to measure the Fresnel reflection by averaging the measured resultant reflection in order to measure the refractive index of the liquid sample. Additionally or alternatively, the detector is configured to measure the scattering by filtering or quantifying or digitally processing the measured resultant reflection in order to measure the turbidity of the liquid sample.

For example, the detector may comprise a photodetector to detect the light signal received from the optical structure. For example, the photodetector may be a silicon, InGaAs, or GaAs based laser power photodetector, which may facilitate a fast response and less sensitivity to the temperature.

For example, the coherent light source may be a laser light source, e.g., a laser diode.

According to a second aspect of the invention, a method for simultaneously measuring refractive index and turbidity of a liquid sample is provided. The method comprises the steps of generating a coherent light signal, transmitting the coherent light signal to the liquid sample, receiving a light signal from the liquid sample in response to the coherent light signal, and measuring refractive index and turbidity of the liquid sample simultaneously from the light signal.

Preferably, the method further comprises the step of measuring or estimating a concentration of dissolved solids in the liquid sample from the refractive index measurement. Additionally or alternatively, the method further comprises the step of measuring or estimating a concentration of undissolved solids in the liquid sample from the turbidity measurement.

It is to be noted that the method according to the second aspect corresponds to the system according to the first aspect and its implementation forms. Accordingly, the method of the second aspect may have corresponding implementation forms. Further, the method of the second aspect achieves the same advantages and effects as the system of the first aspect and its respective implementation forms.

Exemplary embodiments of the invention are now further explained with respect to the drawings by way of example only, and not for limitation. In the drawings:

Fig. 1    shows a first exemplary embodiment of the system according to the first aspect of the invention;

Fig. 2    shows an exemplary embodiment of the detector;

Fig. 3     shows a second exemplary embodiment of the system according to the first aspect of the invention;

Fig. 4     shows a third exemplary embodiment of the system according to the first aspect of the invention;

Fig. 5     shows a fourth exemplary embodiment of the system according to the first aspect of the invention;

Fig. 6     shows a fifth exemplary embodiment of the system according to the first aspect of the invention;

Fig. 7     shows an exemplary electric field distribution in the liquid sample due to reflection and scattering;

Fig. 8     shows exemplary photodetector signals from two Formazin solutions;

Fig. 9     shows exemplary photodetector signals from two Formazin solutions for modulated light sources;

Fig. 10     shows an exemplary graph of turbidity vs USG for human urine samples; and

Fig. 11     shows an exemplary embodiment of the method according to the second aspect of the invention.

Reference will now be made in detail to the embodiments of the present invention, examples of which are illustrated in the accompanying drawings. However, the following embodiments of the present invention may be variously modified and the range of the present invention is not limited by the following embodiments.

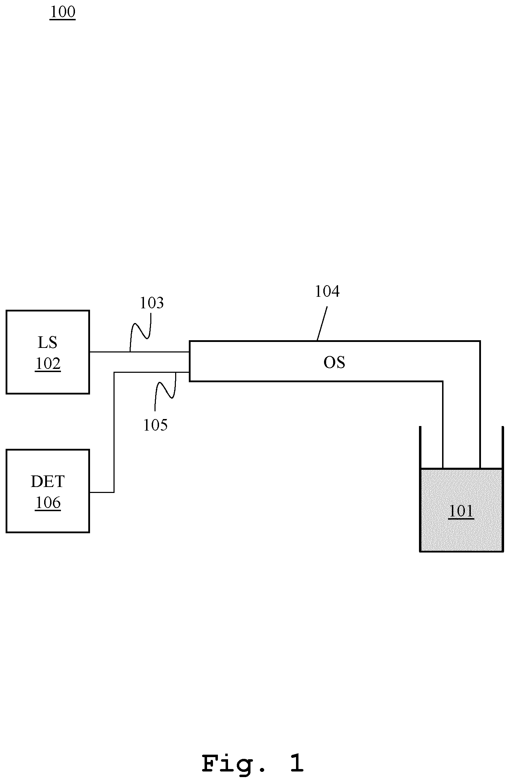

In Fig. 1, a first exemplary embodiment of the system 100 according to the first aspect of the invention is illustrated. The system 100 may simultaneously measure refractive index and turbidity of a liquid sample 101, i.e., may simultaneously act as a refractometer and a turbidimeter.

In this regard, the system 100 may comprise a coherent light source 102 that may generate a coherent light signal 103. For example, the coherent light source 102 may be a laser light source, e.g., a laser diode, which may generate laser beams at a wavelength of 1550 nm.

In addition, the system 100 may comprise an optical structure 104 operably coupled, e.g., optically coupled, to the coherent light source 102 in order to receive and to transmit the coherent light signal 103 through the length of the optical structure 104.

The optical structure 104 may be further in contact with the liquid sample 101, e.g., an end of the optical structure 104 opposite to the end receiving the coherent light signal 103 may be optically coupled with or be immersed in the liquid sample 101.

As such, the optical structure 104 may transmit the coherent light signal 103 to the liquid sample 101 and may receive a return light signal 105, e.g., a back reflected and/or scattered light signal, from the liquid sample 101 in response to the coherent light signal 103.

For example, the return light signal 105 may comprise one or more lights reflected at a surface of the liquid sample 101 in response to the coherent light signal 103 and/or one or more lights scattered within the liquid sample 101 in response to the coherent light signal 103. In this regard, the back reflected and/or scattered lights may be in the same spatial mode of the coherent light signal 103.

The system 100 may further comprise a detector 106 operably coupled, e.g., optically coupled, to the optical structure 104. In this regard, the optical structure 104 may transmit the return light signal 105 from the liquid sample 101 to the detector 106, and the detector may receive and/or detect the return light signal 105 from the optical structure 104.

For instance, the detector 106 may simultaneously measure refractive index and turbidity of the liquid sample 101 from the return light signal 105. In other words, the detector 106 may simultaneously perform a refractive index measurement and a turbidity measurement of the liquid sample 101 from the return light signal 105.

For example, the detector 106 may measure or estimate a concentration of dissolved solids in the liquid sample 101, especially from the refractive index measurement. In addition, the detector 106 may measure or estimate a concentration of undissolved solids in the liquid sample 101 from the turbidity measurement.

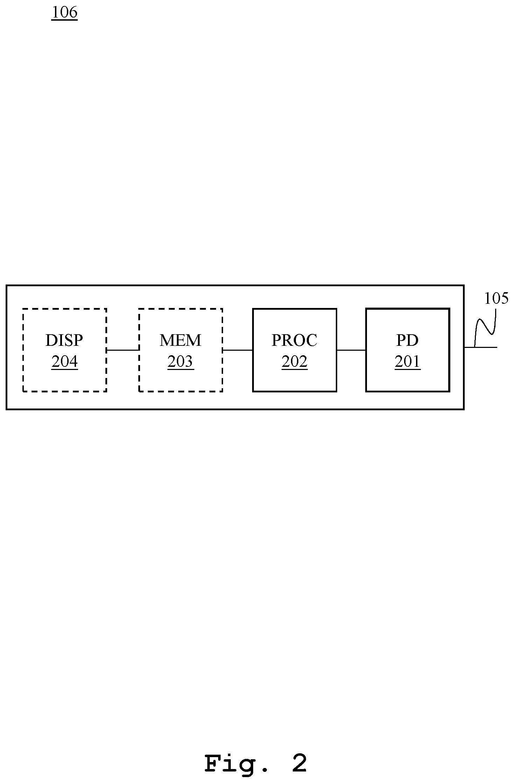

In Fig. 2, an exemplary embodiment of the detector 106 is illustrated. The detector 106 may comprise a photodetector 201, e.g., a photodiode, which may detect the return light signal 105 from the optical structure 104. The detector 106 may further comprise a processor 202 operably coupled to the photodetector 201 and may receive photodetector signals, e.g., photodetector voltage, therefrom.

The processor 202 may process the photodetector signals to perform the refractive index and turbidity measurements of the liquid sample 101, and may correlate the measurements to estimate the concentrations of dissolved and undissolved solids in the liquid sample 101.

For example, the processor 202 may comprise one or more application specific integrated circuits (ASICs), digital signal processors (DSPs), digital signal processing devices (DSPDs), programmable logic devices (PLDs), field programmable gate arrays (FPGAs), controllers, microcontrollers, microprocessors, or the like.

The detector 106 may optionally comprise a memory 203 operably coupled to the processor 202 and/or to the photodetector 201. The memory 203 may store the measurement data from the photodetector 201 and/or the processed measurement data from the processor 202 and/or user instructions for the processor 202.

For example, the memory 203 may comprise random access memory (RAM) units such as static RAM (SRAM) units and dynamic RAM (DRAM) units, register file units, content addressable memory (CAM) units, read only memory (ROM) units, programmable read only memory (PROM) units, removable memory units such as flash memory devices, or the like.

The detector 106 may optionally comprise a display unit or display 204 operably coupled to the memory 203 and/or to the processor 202. The display 204 may allow a user to view the stored measurement data in the memory 203 and/or the processed measurement data from the processor 202, especially in the form of text and/or graphics.

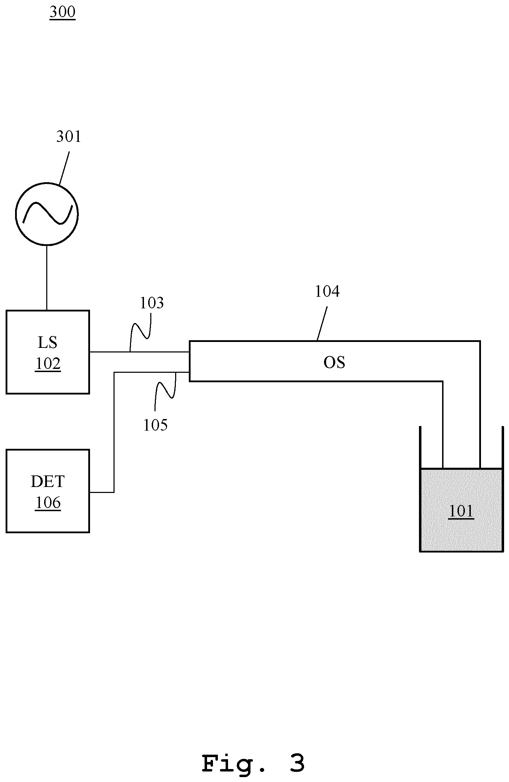

In Fig. 3, a second exemplary embodiment of the system 300 according to the first aspect of the invention is illustrated. The system 300 differs from the system 100 in that the system 300 may additionally comprise a signal generator 301 operably coupled to the coherent light source 102. For example, the signal generator 301 may comprise or be a sine wave generator that may modulate the coherent light source 102 at a predefined frequency, especially to generate a modulated coherent light signal.

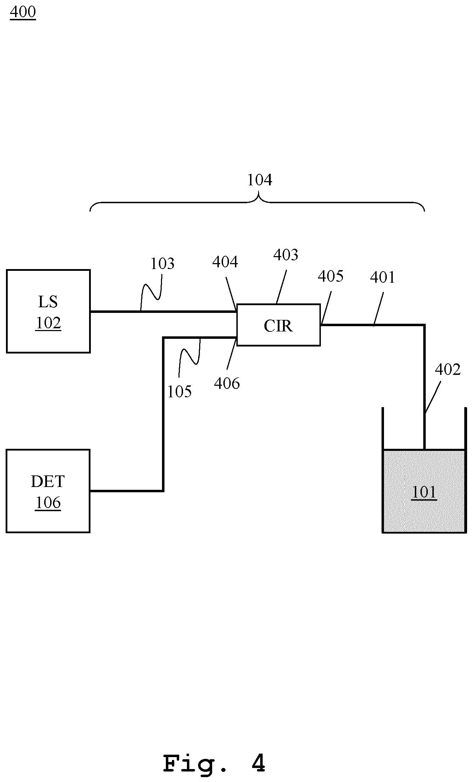

In Fig. 4, a third exemplary embodiment of the system 400 according to the first aspect of the invention is illustrated. The system 400 differs from the system 100 in that the optical structure 104 of the system 400 may comprise a single optical fiber 401 and an optical circulator 403. For example, the optical circulator 403 may be operably coupled, e.g., optically coupled, to the coherent light source 102 and to the detector 106. In addition, an end 402 of the single optical fiber 401 may be immersed in the liquid sample 101.

For instance, the optical circulator 403 may be a three-port optical circulator, especially comprising a first port 404, a second port 405 and a third port 406. In this regard, the optical circulator 403 may be arranged with the single optical fiber 401 such that the coherent light signal 103 may be transmitted from the first port 404 to the second port 405 and further to the liquid sample 101, especially through the single optical fiber 401.

Accordingly, the return light signal 105 may be back transmitted from the second port 405 to the third port 406 and further to the detector 106, especially through the single optical fiber 401. In this regard, the optical circulator 403 may be a single mode optical circulator and/or the single optical fiber 401 may be a single mode optical fiber.

Although not shown, the system 400 may additionally comprise the signal generator 301 of Fig. 3 in order to modulate the coherent light source 102.

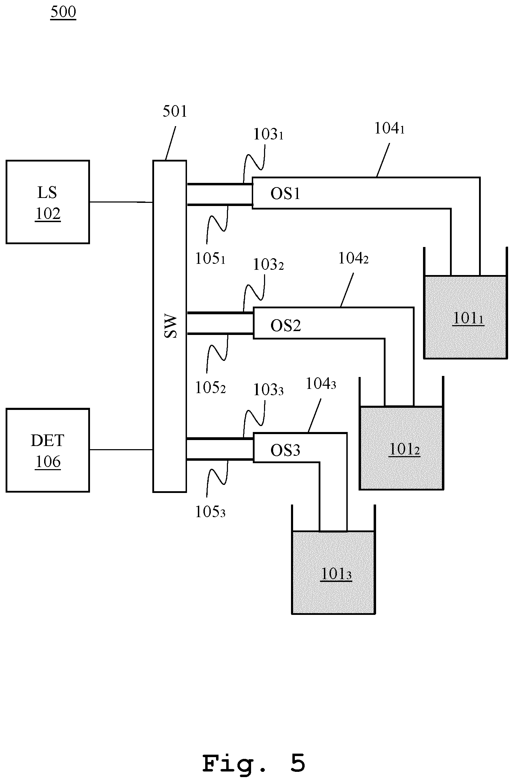

In Fig. 5, a fourth exemplary embodiment of the system 500 according to the first aspect of the invention is illustrated. The system 500 differs from the system 100 in that the system 500 may comprise an optical switch 501 and a plurality of optical structures, exemplarily a first optical structure $104_1$, a second optical structure $104_2$, and a third optical structure $104_3$. It should be understood that the number of optical structures can be more than or less than three, as exemplified herein.

For example, the optical switch 501 may be operably coupled, e.g., optically coupled, to the coherent light source 102 and further to the detector 106. In this regard, the optical switch 501 may receive a first coherent light signal $103_1$, a second coherent light signal $103_2$, and a third coherent light signal $103_3$ from the coherent light source 102, especially in a time-multiplexed manner.

For example, the first coherent light signal $103_1$, the second coherent light signal $103_2$, and the third coherent light signal $103_3$ may be identical signals and may correspond to the coherent light signal 103 of the systems 100, 300, and 400. Alternatively, the first coherent light signal $103_1$, the second coherent light signal $103_2$, and the third coherent light signal $103_3$ may be different signals, e.g., in terms of power and/or phase.

For instance, the first optical structure $104_1$ may be operably coupled, e.g., optically coupled, to the optical switch 501 in order to receive and to transmit the first coherent light signal $103_1$ through the length of the first optical structure $104_1$. The first optical structure $104_1$ may be further in contact with a first liquid sample $101_1$, e.g., an end of the first optical structure $104_1$ opposite to the end receiving the first coherent light signal $103_1$ may be optically coupled with or be immersed in the first liquid sample $101_1$.

As such, the first optical structure $104_1$ may transmit the first coherent light signal $103_1$ to the first liquid sample $101_1$ and may receive a first return light signal $105_1$, e.g., a back reflected and/or scattered light signal, from the first liquid sample $101_1$ in response to the first coherent light signal $103_1$. The back reflected and/or scattered lights may be in the same spatial mode of the first coherent light signal $103_1$.

Similarly, the second optical structure $104_2$ may be operably coupled, e.g., optically coupled, to the optical switch 501 in order to receive and to transmit the second coherent light signal $103_2$ through the length of the second optical structure $104_2$. The second optical structure $104_2$ may be further in contact with a second liquid sample $101_2$, e.g., an end of the second optical structure $104_2$ opposite to the end receiving the second coherent light signal $103_2$ may be optically coupled with or be immersed in the second liquid sample $101_2$.

As such, the third optical structure $104_3$ may transmit the third coherent light signal $103_2$ to the second liquid

sample $101_2$ and may receive a second return light signal $105_2$, e.g., a back reflected and/or scattered light signal, from the second liquid sample $101_2$ in response to the second coherent light signal $103_2$. The back reflected and/or scattered lights may be in the same spatial mode of the second coherent light signal $103_2$.

Similarly, the third optical structure $104_3$ may be operably coupled, e.g., optically coupled, to the optical switch 501 in order to receive and to transmit the third coherent light signal $103_3$ through the length of the third optical structure $104_3$. The third optical structure $104_3$ may be further in contact with a third liquid sample $101_3$, e.g., an end of the third optical structure $104_3$ opposite to the end receiving the third coherent light signal $103_3$ may be optically coupled with or be immersed in the third liquid sample $101_3$.

As such, the third optical structure $104_3$ may transmit the third coherent light signal $103_3$ to the third liquid sample $101_3$ and may receive a third return light signal $105_3$, e.g., a back reflected and/or scattered light signal, from the third liquid sample $101_3$ in response to the third coherent light signal $103_3$. The back reflected and/or scattered lights may be in the same spatial mode of the third coherent light signal $103_3$.

For example, the optical switch 501 may transmit the first return light signal $105_1$, the second return light signal $105_2$, and the third return light signal $105_3$ to the detector 106, especially in the time-multiplexed manner. In other words, the detector 106 may receive and/or detect the first return light signal $105_1$, the second return light signal $105_2$, and the third return light signal $105_3$ in the time-multiplexed manner via the optical switch 501.

Accordingly, the detector 106 may simultaneously measure refractive index and turbidity of the first liquid sample $101_1$ from the first return light signal $105_1$, may simultaneously measure refractive index and turbidity of the second liquid sample $101_2$ from the second return light signal $105_2$, and may simultaneously measure refractive index and turbidity of the third liquid sample $101_3$ from the third return light signal $105_3$.

Although not shown, the system 500 may additionally comprise the signal generator 301 of Fig. 3 in order to modulate the coherent light source 102. In this regard, the first coherent light signal $103_1$, the second coherent light signal $103_2$, and the third coherent light signal $103_3$ may be modulated at different frequencies.

Although not shown, each of the first optical structure $104_1$, the second optical structure $104_1$, and the third optical structure $104_3$ may comprise a single optical fiber 401 and an optical circulator 403, e.g., as shown in Fig. 4 for the system 400.

Although not shown, each the first optical structure $104_1$, the second optical structure $104_1$, and the third optical structure $104_3$ may be in contact with a common liquid sample 101, e.g., as shown in Fig. 1 for the system 100. In this regard, the end of the first optical structure $104_1$, the end of the second optical structure $104_1$, and the end of the third optical structure $104_3$ may be immersed in the liquid sample 101 at different locations, e.g., spatial surface locations.

In this regard, at a first time instant, the detector 106 may simultaneously measure refractive index and turbidity of the liquid sample 101 from the first return light signal $105_1$. Furthermore, at a second time instant, the detector 106 may simultaneously measure refractive index and turbidity of the liquid sample 101 from the second return light signal $105_2$. Moreover, at a third time instant, the detector 106 may simultaneously measure refractive index and turbidity of the liquid sample 101 from the third return light signal $105_3$.

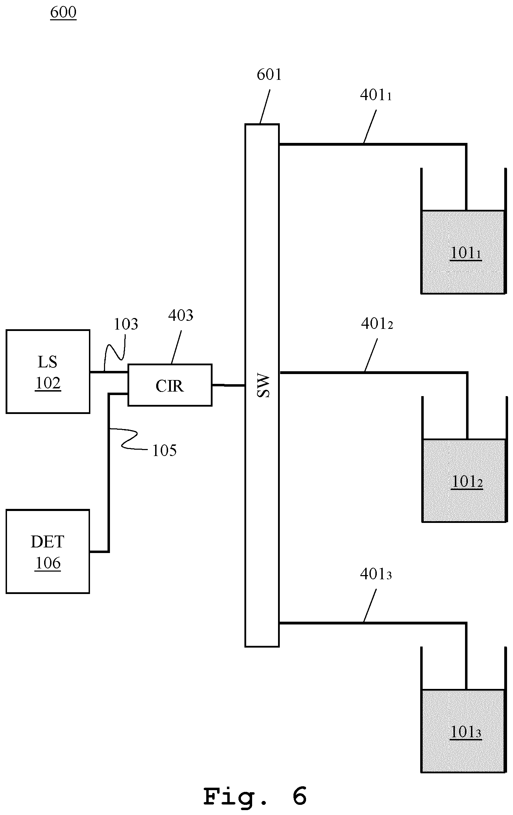

In Fig. 6, a fifth exemplary embodiment of the system 600 according to the first aspect of the invention is illustrated. The system 600 differs from the system 500 in that the system 600 may comprise a single optical circulator 403 optically coupled to an optical switch 601, which is further optically coupled to a plurality of optical fibers, especially single mode optical fibers, exemplarily a first optical fiber $401_1$, a second optical fiber $401_2$, and a third optical fiber $401_3$. It should be understood that the number of optical fibers can be more than or less than three, as exemplified herein.

For example, the optical circulator 403 may be optically coupled to the coherent light source 102 and the detector 106, and further to the optical switch 601, which may correspond to the optical switch 501. In other words, the optical switch 601 may be a single-pole-multiple-throw switch, where the optical circulator 403 may be coupled to the pole port of the optical switch 601 and the first optical fiber $401_1$, the second optical fiber $401_2$, and the third optical fiber $401_3$ may be respectively coupled to the throw ports of the optical switch 601.

In this regard, the optical circulator 403 may receive the coherent light signal 103 from the coherent light source 102, and may transmit the coherent light signal 103 to the first optical fiber $401_1$, the second optical fiber $401_2$, and the third optical fiber $401_3$ via the optical switch 601, especially in a time-multiplexed manner. For example, the coherent light signal 103 may correspond to the coherent light signal 103 of the systems 100, 300, and 400.

For instance, during a first timing operation of the optical switch 601, the first optical fiber $401_1$ may receive and may transmit the coherent light signal 103 through the length of the first optical fiber $401_1$. The first optical fiber $401_1$ may be further in contact with a first liquid sample $101_1$, e.g., an end of the first optical fiber $401_1$ opposite to the end receiving the coherent light signal 103 may be optically coupled with or be immersed in the first liquid sample $101_1$.

Accordingly, the first optical fiber $401_1$ may transmit the coherent light signal 103 to the first liquid sample $101_1$ and may receive the return light signal 105, e.g., a back reflected and/or scattered light signal, from the first liquid sample $101_1$ in response to the coherent light signal 103. The back reflected and/or scattered lights may be in the same spatial mode of the coherent light signal 103.

Similarly, during a second timing operation of the optical switch 601, the second optical fiber $401_2$ may receive

and may transmit the coherent light signal 103 through the length of the second optical fiber $401_2$. The second optical fiber $401_2$ may be further in contact with a second liquid sample $101_2$, e.g., an end of the second optical fiber $401_2$ opposite to the end receiving the coherent light signal 103 may be optically coupled with or be immersed in the second liquid sample $101_2$.

Accordingly, the second optical fiber $401_2$ may transmit the coherent light signal 103 to the second liquid sample $101_2$ and may receive the return light signal 105, e.g., a back reflected and/or scattered light signal, from the second liquid sample $101_2$ in response to the coherent light signal 103. The back reflected and/or scattered lights may be in the same spatial mode of the coherent light signal 103.

Similarly, during a third timing operation of the optical switch 601, the third optical fiber $401_3$ may receive and may transmit the coherent light signal 103 through the length of the third optical fiber $401_3$. The third optical fiber $401_3$ may be further in contact with a third liquid sample $101_3$, e.g., an end of the third optical fiber $401_3$ opposite to the end receiving the coherent light signal 103 may be optically coupled with or be immersed in the third liquid sample $101_3$.

Accordingly, the third optical fiber $401_3$ may transmit the coherent light signal 103 to the third liquid sample $101_3$ and may receive the return light signal 105, e.g., a back reflected and/or scattered light signal, from the third liquid sample $101_3$ in response to the coherent light signal 103. The back reflected and/or scattered lights may be in the same spatial mode of the coherent light signal 103.

For example, the optical circulator 403 may receive the return light signal 105 from the first optical fiber $401_1$, the second optical fiber $401_2$, and the third optical fiber $401_3$ via the optical switch 501, and may accordingly transmit the return light signal 105 to the detector 106, especially in the time-multiplexed manner.

For example, the detector 106 may simultaneously measure refractive index and turbidity of the first liquid sample $101_1$ from the return light signal 105 during the first timing operation of the optical switch 601, may simultaneously measure refractive index and turbidity of the second liquid sample $101_2$ from the return light signal 105 during the second timing operation of the optical switch 601, and may simultaneously measure refractive index and turbidity of the third liquid sample $101_3$ from the return light signal 105 during the third timing operation of the optical switch 601.

Although not shown, the system 500 may additionally comprise the signal generator 301 of Fig. 3 in order to modulate the coherent light source 102. In this regard, the coherent light signal 103 may be modulated at different frequencies.

Although not shown, each the first optical fiber $401_1$, the second optical fiber $401_2$, and the third optical fiber $401_3$ may be in contact with a common liquid sample 101, e.g., as shown in Fig. 1 for the system 100. In this regard, the end of the first optical fiber $401_1$, the end of the second optical fiber $401_2$, and the end of the third optical fiber $401_3$ may be immersed in the liquid sample 101 at different locations, e.g., spatial surface locations.

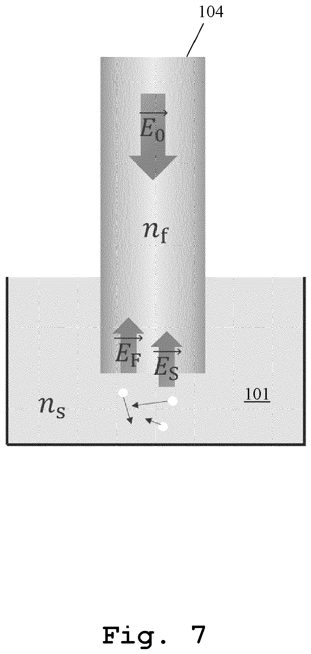

In Fig. 7, an exemplary electric field distribution in the liquid sample 101 due to reflection and scattering is illustrated. As shown in Fig. 7, the optical structure 104, especially an end of the optical structure 104, may be immersed in the liquid sample 101. In this example, $n_f$ denotes the refractive index of the optical structure 104 and $n_s$ denotes the refractive index of the liquid sample 101.

Moreover, an electric field $E_0$ may denote the electric field of the coherent light signal 103, which may travel along the optical structure 104 with the refractive index $n_f$, and may be incident on the surface of the liquid sample 101 with the refractive index $n_s$. In this regard, most of the incident light may enter into the liquid sample 101, however due to the mismatch of the refractive index of the liquid sample 101 and of the optical structure 104, a small portion may reflect at the interface.

For example, an electric field $E_F$ may denote the electric field of said reflection. In addition, further differences in refractive indices coming from structures in the liquid may cause further reflections where part of the light from these further reflections may cross back over the interface. For example, an electric field of said further reflections or scattering may be denoted by an electric field $E_S$.

The detector 106 may measure the light directly reflected from the liquid sample 101 due to the illumination of the liquid sample 101 by the coherent light signal 103. The term "directly reflected light" should be understood as the light that may be reflected in the same spatial mode as the incident coherent light signal 103, i.e., 180 degrees to the incident coherent light signal 103.

For example, due to the illumination of the liquid sample 101, a Fresnel reflection with the electric field $E_F$ may occur at the interface, e.g., due to the difference between the refractive indices of the liquid sample 101 and the optical structure 104. Furthermore, the amount of scattering of the light that may enter the liquid sample 101 may be dependent on the turbidity, which can be understood as the scattering of the light depending on the undissolved solids or colloids or suspended bubbles or unmixed liquid region. A part of this scattering of the light with the electric field $E_s$ may be directed in the same direction as the Fresnel reflection.

Accordingly, the resultant overall reflection $E_R$ can be written as:

$$\vec{E_R} = \vec{E_F} + \vec{E_S} \tag{1}$$

As such, the light due to the Fresnel reflection and the light from scattering may interfere, especially due to the coherence length of the light, e.g., approximately the same size as the distance between the interface and the scattering particles, or greater. Accordingly, due to the constant motion of the scattering particles in the liquid sample 101, the detector 106 may measure the overall reflection as a constant with fluctuations around said value due to the scattering.

For example, the electric fields can be expressed as sinusoidal plane waves as a function of time t in one spatial dimension z in the following manner:

$$E_R = E_F + E_S = A_F \cos(kz - \omega t) + A_S \cos(kz - \omega t + \varphi) \tag{2}$$

where k is the wavenumber, $\omega$ is the angular velocity, $A_F$ is the amplitude of the Fresnel reflected light, $A_S$ is the amplitude of the scattered light, and $\varphi$ is a phase shift.

Due to the sensitivity of the photodetectors, such as the photodetector 201, to the light power rather than the electric field, the reflected power $P_R$ can be written in the following manner:

$$P_R \propto E_R^2 =$$
$$A_F^2 \cos^2(-\omega t) + A_S^2 \cos^2(-\omega t + \varphi) + 2A_F A_S \cos(-\omega t)\cos(-\omega t + \varphi) \tag{3}$$

where the position of the photodetector is defined to be at z=0.

Using a suitable trigonometric identity, equation (3) can be written in the following manner:

$$P_R \propto E_R^2 =$$
$$A_F^2 \cos^2(-\omega t) + A_S^2 \cos^2(-\omega t + \varphi) + A_F A_S(\cos(\varphi) + \cos(-2\omega t + \varphi)) \tag{4}$$

Furthermore, the terms that oscillate with an angular frequency of $\omega$ or $2\omega$ can be replaced with their time average values, e.g., due to the fast oscillation that may not be detectable by the photodetector, in the following manner:

$$P_R \propto E_R^2 = \frac{A_F^2 + A_S^2}{2} + A_F A_S \cos(\varphi) \tag{5}$$

In case the contribution from scattering is too small, i.e., $A_F \gg A_S$, equation (5) can be reduced in the following manner:

$$P_R \propto \frac{A_F^2}{2} + A_F A_S \cos(\varphi) \tag{6}$$

As it can be seen from equation (6), the first term is constant and may depend only on the Fresnel reflection. In the second term, **cos($\varphi$)** can take any value between -1 and 1, and is symmetrical around 0. The phase shift $\varphi$ is dependent on the position of the surfaces of the scattering particles, and may cause the fluctuations due to the constant movement of the scattering particles. In addition, the amplitude $A_S$ may be dependent on the turbidity, which may increase as the turbidity increases. Furthermore, the measurement of the Fresnel reflection can give a measurement of the refractive index of the liquid sample 101.

Therefore, from equation (6), it is possible to measure the contribution from the Fresnel reflection independently of the turbidity. For example, the simple average of the measured signal may result in the contribution only from the Fresnel reflection, and hence may give a measure of the refractive index. On the other hand, a quantification, e.g., high-pass filtration followed by a root-mean-square operation, or a suitable autocorrelation function, of the fluctuations may only correlate with the turbidity.

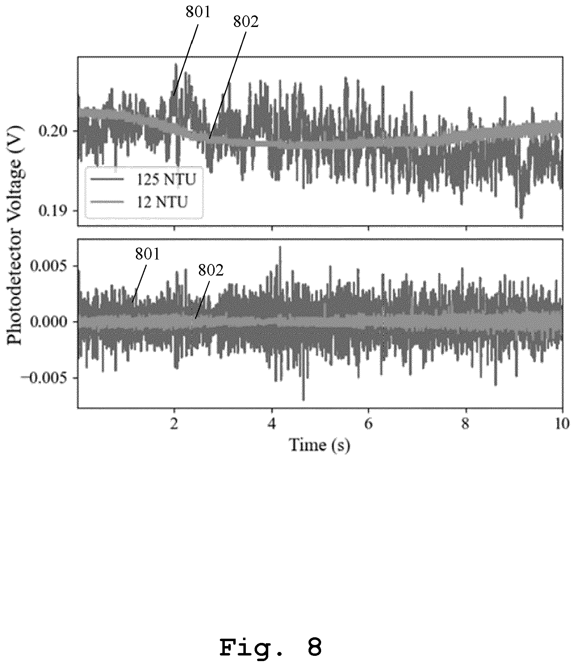

In Fig. 8, exemplary photodetector signals from two Formazin solutions 801, 802 are illustrated. The horizontal axis denotes the time in seconds and the vertical axis denotes the photodetector voltage in volts. The turbidity value for the first Formazin solution 801 is measured as 125 NTU and the turbidity value for the second Formazin solution 802 is measured as 12 NTU. The top plot corresponds to the raw photodetector signals from the Formazin solutions. The bottom plot corresponds to the same signals as the top plot, however, after applying a high-pass filter.

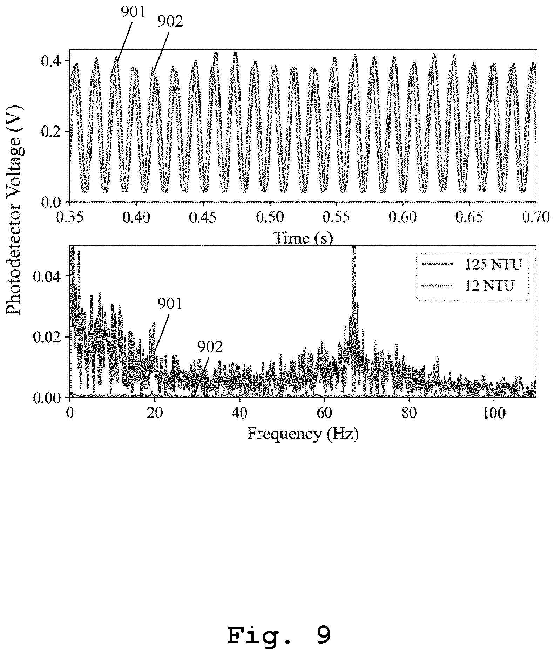

In Fig. 9, exemplary photodetector signals from two Formazin solutions 901, 902 for modulated light sources are illustrated. Particularly for a modulated light source, the signal level corresponding to the Fresnel reflection can be retrieved through a Fourier transform. In this regard, the value at the modulation frequency may be related to the Fresnel reflection, whereas the power at other frequencies may be related to the amount of turbidity.

Turning back to Fig. 9, the horizontal axis of the top plot denotes the time in seconds and the vertical axis of the top plot denotes the photodetector voltage in volts. Additionally, the horizontal axis of the bottom plot denotes the frequency in Hz and the vertical axis of the bottom plot denotes the photodetector voltage in volts. In this regard, the light source, i.e., laser, is modulated at a frequency of 67 Hz.

The top plot corresponds to the raw photodetector signals from the Formazin solutions 901, 902, which are identical to the Formazin solutions 801, 802 of Fig. 8. The bottom plot corresponds to the same signals as the top plot, however after applying a Fourier transform.

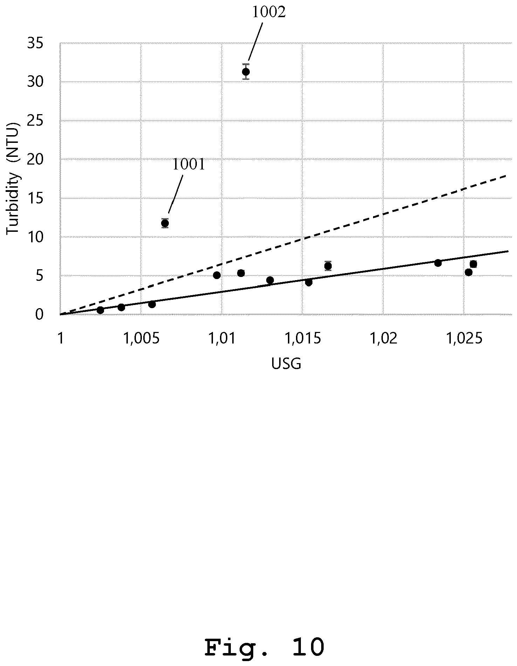

In Fig. 10, an exemplary graph of turbidity vs Urine specific gravity (USG) for human urine samples is illustrated.

For example, the liquid sample 101 may be a urine sample, and the concentration of dissolved solids in the liquid sample is a concentration of dissolved metabolites in the urine sample. In addition, the concentration of undissolved solids in the liquid sample is the concentration of secreted proteins in the urine sample.

An increased level of protein in urine (Proteinuria) may be a sign of disease. This may be due to an increase in the protein secreted into the urine, which would normally be filtered by the kidneys. However, a simple measurement of the protein level, e.g., with a spot check, may give false negatives of Proteinuria if the urine is diluted. Therefore, a clinical diagnosis may be required by taking urine over a 24-hour period and by measuring the average protein concentration.

However, a spot check would be of more value for preventative healthcare purposes, especially since in early stages, it might be possible to detect an increase in protein without symptoms. In principle, this spot check may need to measure the protein concentration while factoring out the effect of urine dilution, i.e., a combined measurement of protein and urine specific gravity (USG).

Therefore, in the case of the urine sample, the detector 106 may measure the urine turbidity to estimate the undissolved portion in the urine sample, thereby measuring the protein content and may further measure the refractive index to estimate the dissolved metabolites in the urine sample, thereby measuring the USG.

In particular, the detector 106 may perform the turbidity and refractive index measurement simultaneously, especially to simultaneously measure the secreted protein and USG, and may use their ratio as a possible parameter to yield proteinuria diagnosis.

Turning back to Fig. 10, the horizontal axis denotes USG and the vertical axis denotes turbidity in nephelometric turbidity unit (NTU). The solid line represents the normal turbidity level for a given USG and the dashed line represents the level at which proteinuria can be declared. It is to be noted that the lines can also be formed as curves or other complex functions. It can be seen that two samples 1001, 1002 are abnormal and result in a much higher turbidity to USG ratio.

Alternatively, for example, the liquid sample may be a bioreactor sample and the concentration of dissolved solids in the liquid sample is a concentration of a substrate in the bioreactor sample. Additionally, the concentration of undissolved solids in the liquid sample is a concentration of cells in suspension in the bioreactor sample.

For example, in optimal conditions, the rate of cell growth in a bioreactor can be expressed by the Monod equation for cell growth in the following manner:

$$r = \mu_{\max} \frac{xS}{K+S} \qquad\qquad (7)$$

where r is the volumetric rate of cell growth, $\mu_{max}$ is the maximum specific growth rate, K is the saturation constant, x is the cell concentration and S is the substrate concentration, e.g., the concentration of nutrient or food for cells.

In this regard, during the process, the variables are x and S, while the other terms are constants. Therefore, measurements of x and S may result in a calculation of the optimal growth rate. If the measured actual growth rate, i.e., the change in x over time, is significantly lower than the calculated optimal growth rate, this could act as an early warning for process control.

Furthermore, the cells in suspension may increase the turbidity of the liquid, which may result in the measurement of cell density. In addition, the cell growth substrate may consist of dissolved solids, such as sugars, which may alter the refractive index. This may result in the measurement of the substrate concentration.

Therefore, in the case of the bioreactor sample, the detector 106 may measure the turbidity to estimate the undissolved solids, i.e., the cells in suspension, in the bioreactor sample, thereby measuring the cell density and may further measure the refractive index to estimate the dissolved solids, e.g., sugars, in the bioreactor sample, thereby measuring the substrate concentration. Accordingly, the optimal growth rate can be calculated with simultaneous measurements of turbidity and refractive index.

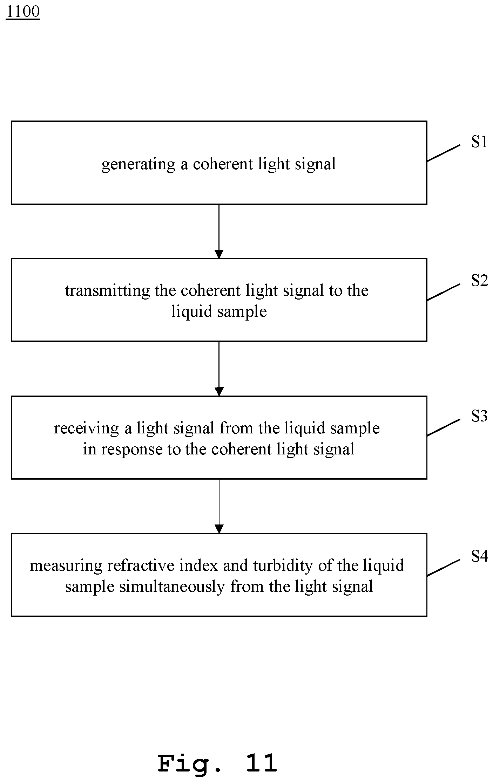

In Fig. 11, an exemplary embodiment of the method 1100 according to the second aspect of the invention is illustrated. In a first step S1, a coherent light signal is generated. In a second step S2, the coherent light signal is transmitted to the liquid sample. In a third step S3, a light signal is received from the liquid sample in response to the coherent light signal. In a fourth step S4, refractive index and turbidity of the liquid sample are simultaneously measured from the light signal.

Therefore, the invention facilitates the measurement or estimation of undissolved solids in reference to the dissolved solids or vice versa in a liquid sample, especially through the measurement of back reflected and/or scattered coherent light. The amount of fluctuation in the measured signal may correlate with turbidity, while the average signal level may correlate with the Fresnel reflection, which may result in simultaneous measurements of turbidity and refractive index. It is to be noted that the measurement of refractive index can be understood as the measurement of refractive index of the liquid sample between the particles that cause turbidity.

Furthermore, turbidity may be caused by the presence of the undissolved solids while the refractive index may be changed by the dissolved solids, which may allow for the simultaneous measurement or estimation of the quantity of these two types of solids in the liquid sample.

It is important to note that, in the description as well as in the claims, the word "comprising" does not exclude other elements or steps and the indefinite article "a" or "an" does not exclude a plurality. A single element or other unit may fulfill the functions of several entities or items recited in the claims. It should be understood that the term "and/or" used in the specification and the appended claims of this application refers to any combination and all possible combinations of one or more associated listed items, and includes these combinations.

It should also be understood that the word "coupled" implies that the elements may be directly connected together or may be coupled through one or more intervening elements. Moreover, the disclosure with regard to any of the aspects is also relevant with regard to the other aspects of the disclosure.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

**Claims**

1.  A system (100, 300, 400, 500, 600) for simultaneously measuring refractive index and turbidity of a liquid sample (101), the system comprises:

    a coherent light source (102) configured to generate a coherent light signal (103),
    an optical structure (104) being in contact with the liquid sample (101), the optical structure (104) is configured to transmit the coherent light signal (103) to the liquid sample (101) and further to receive a light signal (105) from the liquid sample (101) in response to the coherent light signal (103), and
    a detector (106) configured to receive the light signal (105) and further to measure refractive index and turbidity of the liquid sample (101) simultaneously from the light signal (105).

2.  The system according to claim 1,

    wherein the detector (106) is further configured to measure or estimate a concentration of dissolved solids in the liquid sample (101) from the refractive index measurement, and/or
    wherein the detector (106) is further configured to measure or estimate a concentration of undissolved solids in the liquid sample (101) from the turbidity measurement.

3.  The system according to claim 2,

    wherein the concentration of dissolved solids in the liquid sample (101) is a concentration of dissolved metabolites in a urine sample, and
    wherein the concentration of undissolved solids in the liquid sample (101) is a concentration of secreted proteins in the urine sample.

4.  The system according to claim 2,

    wherein the concentration of dissolved solids in the liquid sample (101) is a concentration of a substrate in a bioreactor, and
    wherein the concentration of undissolved solids in the liquid sample (101) is a concentration of cells in suspension in the bioreactor.

5. The system according to any of claims 1 to 4,
   wherein the light signal (105) comprises one or more lights reflected at a surface of the liquid sample (101) and/or one or more lights scattered within the liquid sample (101), especially in response to the coherent light signal (103).

6. The system according to any of claims 1 to 5,
   wherein the optical structure (104) comprises a single optical fiber (401) configured to transmit the coherent light signal (103) to the liquid sample (101) and further to receive the light signal (105) from the liquid sample (101) .

7. The system according to claim 6,
   wherein the single optical fiber (401) is a single mode optical fiber.

8. The system according to claim 6 or 7,
   wherein the single optical fiber (401) is configured such that an end (402) of the single optical fiber (401) is immersed in the liquid sample (101).

9. The system according to any of claims 1 to 8,
   wherein the optical structure (104) comprises an optical circulator (403) configured to direct the coherent light signal (103) towards the liquid sample (101) and further to direct the light signal (105) from the liquid sample (101).

10. The system according to any of claims 1 to 9,
    wherein the system further comprises a signal generator (301) configured to modulate the coherent light source (102), preferably at a predefined frequency.

11. The system according to any of claims 1 to 10,

    wherein the system comprises:

    an optical switch (501), and
    a plurality of optical structures ($104_1$-$104_3$),

    wherein the optical switch (501) is configured such that each of the plurality of optical structures ($104_1$-$104_3$) transmits a respective coherent light signal ($103_1$-$103_3$) from the coherent light source (102) to the liquid sample ($101_1$-$101_3$) and further to receive a respective light signal ($105_1$-$105_3$) from the liquid sample ($101_1$-$101_3$) in response to the coherent light signal ($103_1$-$103_3$), and
    wherein the detector (106) is configured to receive the light signals ($105_1$-$105_3$) from the plurality of optical structures ($104_1$-$104_3$) and further to measure refractive index and turbidity of the liquid sample simultaneously from each of the light signals ($105_1$-$105_3$).

12. The system according to any of claims 1 to 11,
    wherein the detector (106) is configured to measure a resultant reflection from the light signal (105) corresponding to an interference between lights due to Fresnel reflection and lights from scattering in the liquid sample (101), especially due to the transmission of the coherent light signal (103) to the liquid sample (101).

13. The system according to claim 12,

    wherein the detector (106) is configured to measure the Fresnel reflection by averaging the measured resultant reflection in order to measure the refractive index of the liquid sample (101), and/or
    wherein the detector (106) is configured to measure the scattering by filtering the measured resultant reflection in order to measure the turbidity of the liquid sample (101) .

14. A method (1100) for simultaneously measuring refractive index and turbidity of a liquid sample, the method comprises:

    generating (S1) a coherent light signal,
    transmitting (S2) the coherent light signal to the liquid sample,
    receiving (S3) a light signal from the liquid sample in response to the coherent light signal, and
    measuring (S4) refractive index and turbidity of the liquid sample simultaneously from the light signal.

15. The method according to claim 14,

wherein the method further comprises:

measuring or estimating a concentration of dissolved solids in the liquid sample from the refractive index measurement, and/or
measuring or estimating a concentration of undissolved solids in the liquid sample from the turbidity measurement.

100

LS
102

103

104

OS

105

DET
106

101

Fig. 1

106

```
DISP      MEM      PROC     PD
204       203      202      201
```

105

Fig. 2

300

301

103    104

LS
102

OS

DET
106

105

101

Fig. 3

400

Fig. 4

500

Fig. 5

<u>600</u>

601    401$_1$

403    103

LS
<u>102</u>

CIR

SW

<u>101$_1$</u>

401$_2$

<u>101$_2$</u>

105

DET
<u>106</u>

401$_3$

<u>101$_3$</u>

Fig. 6

Fig. 7

Fig. 8

Fig. 9

Fig. 10

1100

| generating a coherent light signal | S1 |

| transmitting the coherent light signal to the liquid sample | S2 |

| receiving a light signal from the liquid sample in response to the coherent light signal | S3 |

| measuring refractive index and turbidity of the liquid sample simultaneously from the light signal | S4 |

Fig. 11

Europäisches
Patentamt

European
Patent Office

Office européen
des brevets

# EUROPEAN SEARCH REPORT

**Application Number**

EP 23 21 3193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H11 295220 A (KYOTO DAIICHI KAGAKU KK) 29 October 1999 (1999-10-29) | 1,2,4, 10,14,15 | INV. G01N21/41 |
| Y | * paragraphs [0005], [0016], [0021], | 3 | G01N21/43 |
| A | [0030] - [0034]; figure 1 * | 13 | G01N21/51 |
| | ----- | | G01N21/85 |
| X | KUCHEJDA M: "FIBER OPTIC REFRACTOMETER WITH INHERENT TURBIDITY MEASUREMENT", PROCEEDINGS OF SPIE, IEEE, US, vol. 798, 31 March 1987 (1987-03-31), pages 246-248, XP001030490, ISBN: 978-1-62841-730-2 | 1,2,4-6, 8,10,12, 14,15 | ADD. G01N21/47 |
| Y | * the whole document * | 9 | |
| A | | 7,13 | |
| | & DE 33 02 089 C2 (FRAUNHOFER GES FORSCHUNG [DE]) 17 April 1986 (1986-04-17) * column 2, line 27 - line 59; figure 1 * | | |
| | ----- | | |
| X | DE 34 08 417 C1 (FRAUNHOFER GES FORSCHUNG) 13 December 1984 (1984-12-13) | 1,4,5, 10,12,14 | |
| Y | * page 3, line 15 - line 59; figure 1 * | 11 | |
| A | | 7,13 | TECHNICAL FIELDS SEARCHED (IPC) |
| | ----- | | |
| Y | CN 102 914 519 B (HEFEI INST PHYSICAL SCI CAS ET AL.) 15 April 2015 (2015-04-15) * paragraph [0007]; figure 1 * | 9 | G01N |
| | ----- | | |
| Y | FR 2 899 971 A1 (INST FRANCAIS DU PETROLE [FR]) 19 October 2007 (2007-10-19) * figure 1 * | 11 | |
| | ----- | | |
| Y | US 2015/359522 A1 (RECHT MICHAEL I [US] ET AL) 17 December 2015 (2015-12-17) * paragraphs [0029], [0082], [0085], [0107] - [0109] * | 3 | |
| | ----- | | |

The present search report has been drawn up for all claims

| Place of search | Date of completion of the search | Examiner |
|---|---|---|
| Munich | 11 April 2024 | Weinberger, Thorsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 3193

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2024

| Patent document cited in search report | | Publication date | Patent family member(s) | | Publication date |
|---|---|---|---|---|---|
| JP H11295220 | A | 29-10-1999 | NONE | | |
| DE 3408417 | C1 | 13-12-1984 | NONE | | |
| CN 102914519 | B | 15-04-2015 | NONE | | |
| FR 2899971 | A1 | 19-10-2007 | NONE | | |
| US 2015359522 | A1 | 17-12-2015 | KR 20150144697 A | | 28-12-2015 |
| | | | US 2015359522 A1 | | 17-12-2015 |
| | | | US 2019090859 A1 | | 28-03-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 115236034 A